# EUROPEAN PATENT APPLICATION

(11) **EP 1 952 818 A1**
(43) Date of publication of application: **06.08.2008**
(21) Application number: 06822490.6
(22) Date of filing: 27.10.2006
(51) Int. Cl.: A61K 38/00, A61K 9/127, A61P 1/02, A61P 19/08, A61P 19/10, A61P 29/00, A61P 43/00

(54) **INHIBITOR OF OSTEOCLAST FORMATION, COMPOSITION FOR ORAL ADMINISTRATION AND PROPHYLACTIC OR THERAPEUTIC AGENT FOR BONE DISEASE COMPRISING LACTOFERRIN-CONTAINING LIPOSOME**

(30) Priority: 27.10.2005 JP 2005312607
(71) Applicant: SUNSTAR INC., Takatsuki-shi, Osaka 569-1195 (JP)
(72) Inventor: ISHIKADO,Atsushi, Osaka, 5691195 (JP); IMANAKA, Hiromichi, Osaka, 5691195 (JP); SUIDO, Hirohisa, Osaka, 5691195 (JP); MAKINO, Taketoshi, Osaka, 5691195 (JP)
(74) Representative: Jones, Helen M.M.
(86) International application number: PCT/JP2006/321529
(87) International publication number: WO 2007/049757

(57) **Abstract**

A growth of osteoclasts is inhibited by orally administering a liposomal lactoferrin. Thus a bone disease in which the osteoclast is involved is effectively prevented or treated by orally administering the liposomal lactoferrin. The present invention provides an osteoclast growth inhibitor, an oral composition and a preventive and therapeutic agent for bone diseases (osteoporosis, rheumatoid, periodontal diseases with alveolar bone resorption, which contain the liposomal lactoferrin, and a preventive or therapeutic method for the bone diseases. According to the present invention, the growth of the osteoclasts can be inhibited and the bone diseases due to promotion of the osteoclast growth can be prevented or treated.

## Description

### Technical Field

The present invention relates to an osteoclast growth inhibitor, an oral composition, and a preventive or therapeutic agent for bone diseases, which inhibit a growth of osteoclasts.

### Background Art

A bone tissue is composed of cells constituting the bone and intercellular matrix, and composed mainly of the latter in weight. The intercellular matrix includes collagen fibers and inorganic components. In this bone tissue, remodeling occurs consistently for maintaining the homeostasis in morphological changes and calcium concentrations in blood. In normal adults, an amount of bone resorption and an amount of bone formation are balanced, and thus, a bone weight is scarcely changed. However, when the amount of bone formation corresponding to the amount of bone resorption is not obtained, a bone disease typified by osteoporosis occurs. Classifying factors for such cases in consideration of the bone remodeling, the factors to cause the osteoporosis are classified into the following three: (1) decrease of the bone weight associated with promotion of the bone resorption, typified by osteoporosis in a menopausal early stage and hyperthyroidism, (2) decrease of the bone weight associated with the reduction of bone resorption and bone formation functions, typified by senile osteoporosis and diabetic osteoporosis, and (3) decrease of the bone weight because the bone resorption amount exceeds the bone formation amount in consequence (e.g., shortage of calcium ingestion) although the bone resorption and the bone formation are normal. This way, the osteoporosis increases in occurrence rate with aging, and rapidly increases in postmenopausal women.

Representative methods currently used for the treatment or the prevention of the bone diseases include (1) supplement of calcium/magnesium, (2) administration of calcitonin, (3) employment of therapeutic exercise, (4) administration of active vitamin D (especially targeting the elderly), (5) administration of an estrogen formulation (especially targeting the postmenopausal women) and (6) administration of a parathyroid hormone. Typically, the combination of them is administered in consideration of its cause.

However, among them, the hormone formulation can not be used for the prevention, and even if used for the treatment, a risk to cause side effects is high. Thus, its use is necessary to be strictly controlled by a physician. No sufficient effect is obtained by the other method not combining the hormone therapy. Therefore, development of the method for the treatment or the prevention with no risk of the side effect is desired, and many proposals have been made. Representatives include the proposal which focused on a mineral material whose resorption rate is high and the combination thereof (Patent Documents 1 and 2), the proposal which focused on the combination of a component which enhances the resorption rate of a mineral with the mineral (Patent Documents 3 and 4) and the proposal which focused on a substance which enhances the function of the cells constituting the bone (Patent Documents 5 and 6).
Patent Document 1: JP 1987(S62)-501843-A
Patent Document 2: JP 1994(H06)-500552
Patent Document 3: JP 1994(H06)-40922-A
Patent Document 4: JP 1994(H06)-70726-A
Patent Document 5: JP 1995(H07)-76235-B
Patent Document 6: JP 1990(H02)-303457-A

### DISCLOSURE OF INVENTION

### PROBLEMS TO BE SOLVED BY THE INVENTION

However, even when the above proposals are used, the bone diseases can not be sufficiently prevented or treated, and the proposal having the higher effect is desired. Specifically, it is necessary to balance the bone formation amount and the bone resorption amount in the prevention or the treatment of the above bone diseases. The bone remodeling is primarily controlled by balancing the bone formation and the bone resorption. The osteoclast takes on the bone resorption. It is believed that the bone resorption is regulated by differentiation of the osteoclast, regulation of a bone resorption activity of the osteoclast and apoptosis of the osteoclast. Thus, by inhibiting the growth of the osteoclast, it is possible to inhibit the bone resorption. In the osteoporosis, the bone resorption is increased by an action of the osteoclast to disturb the balance of the bone formation and the bone resorption, thereby decreasing the bone weight to make the bone breakable. In rheumatoid arthritis, the matrix is directly destroyed by various proteases produced by inflammatory synovial membranes, as well as cartilages and the bone are destroyed by the osteoclast. Therefore, by inhibiting the growth of the osteoclast, it becomes possible to prevent or treat the bone diseases including osteoporosis and rheumatoid arthritis, in which the osteoclast is involved.

### MEANS FOR SOLVING PROBLEMS

As a result of an extensive study for solving the above problems, the present inventors have found that a liposome containing lactoferrin has an excellent inhibitory effect on osteoclast growth, and have completed the present invention.

That is, the present invention provides the following osteoclast growth inhibitor, oral composition, preventive or therapeutic agent and preventive or therapeutic method for bone diseases, and use thereof.
[1] An osteoclast growth inhibitor containing a liposomal lactoferrin.
[2] An oral composition containing the osteoclast growth inhibitor according to [1].
[3] A preventive or therapeutic agent for bone diseases containing a liposomal lactoferrin.
[4] The preventive or therapeutic agent according to [3] wherein the bone disease is osteoporosis.
[5] The preventive or therapeutic agent according to [3] wherein the bone disease is rheumatoid.
[6] The preventive or therapeutic agent according to [3] wherein the bone disease is a periodontal disease with alveolar bone resorption.
[7] A preventive or therapeutic method for bone diseases characterized by administering a liposomal lactoferrin in an amount effective for prevention or treatment of bone diseases to a mammalian animal.
[8] The preventive or therapeutic method according to [7] wherein the bone disease is osteoporosis.
[9] The preventive or therapeutic method according to [7] wherein the bone disease is rheumatoid.
[10] The preventive or therapeutic method according to [7] wherein the bone disease is a periodontal disease with alveolar bone resorption.
[11] Use of a liposome comprising lactoferrin for producing a preventive or therapeutic agent for bone diseases.
[12] The use according to [11] wherein the bone disease is osteoporosis.
[13] The use according to [11] wherein the bone disease is rheumatoid.
[14] The use according to [11] wherein the bone disease is a periodontal disease with alveolar bone resorption.

The present application can include the following method for inhibiting the growth of the osteoclast.
[i] A method for inhibiting growth of an osteoclast characterized by administering a liposomal lactoferrin in an effective amount for inhibiting the growth of the osteoclast.

In the present invention, the liposome is a lipid vesicular body having a bimolecular membrane formed by hydrating a lipid composed mainly of a phospholipid with a sufficient amount of water. The liposome can incorporate a water-soluble medicament in its internal aqueous layer and incorporate a lipid-soluble medicament in its lipid bilayer. Thus, its practical application has been attempted as a medicament carrier of a DDS drug for the purpose of targeting of the medicament, making a sustained release drug and reducing the side effect. It is also known that the liposome is highly safe because it is composed of components in a biological membrane.

Generally, the liposome is classified based on a number of the lipid bilayer, and classified into a multilamellar membrane liposome (MLV) and a unilamellar liposome. The unilamellar liposome is further divided into SUV (small unilamella vesicle), LUV (large unilamella vesicle) and GUV (giant unilamella vesicle) depending on its size. The liposome of the present invention may be any of them. Preferable is MLV. In the present invention, the size of the liposome is typically 30 to 1000 nm, preferably 30 to 600 nm and more preferably 50 to 200 nm.

In the liposomal lactoferrin used in the present invention, lactoferrin is preferably encapsulated in a space surrounded by a liposome membrane, but may be included as a liposome membrane constituent, or may be included between multilamellar membranes which compose the multilamellar membrane liposome, or may be included in a form of adhering or binding lactoferrin to an outmost membrane of the liposome membranes.

The liposomal lactoferrin can be produced by conventional methods. For example, the liposomal lactoferrin can be obtained by solubilizing lecithin in a desired amount and if necessary sterol in a desired amount with an appropriate organic solvent such as ethanol, removing the solvent under reduced pressure to make a membrane lipid, subsequently adding an aqueous solution comprising lactoferrin and an optional physiologically active substance thereto and stirring at about 1000 to 3000 rpm for 2 to 5 minutes to prepare a liposome suspension.

Also, separately from this method, the liposomal lactoferrin can also be obtained by dissolving lecithin in the desired amount and if necessary sterol in the desired amount in ethanol in a small amount, dispersing it in an aqueous solution or a buffer to perform preliminary emulsification and dispersing it under high pressure to prepare a liposome suspension.

For the resulting suspension, if necessary the manipulation to remove lactoferrin in the liquid out of the liposomes may be performed. For example, the suspension may be filtrated and the resulting filtrate may be dialyzed.

The liposome suspension can be directly used in a liquid form, and can also be used as a dried product obtained by lyophilizing it. It is possible to make the liposome various forms suitable for oral ingestion, including tablets and capsules made from the dried product.

A source of the lactoferrin used in the present invention is not limited, and for example, it is possible to use lactoferrin obtained from the mammalian animal (human beings, cattle, sheep, goat, horse and the like). Among them, it is preferable that lactoferrin is derived from the human beings or the cattle. Lactoferrin is obtained by separating from milk or milk processed products (e.g., defatted milk, whey) using standard methods. Apolactoferrin obtained by removal of iron from it with hydrochloric acid or citric acid, metal saturated lactoferrin obtained by chelating it with the metal such as iron, copper, zinc or manganese, lactoferrin obtained by saturating the metal at various saturation degrees, or an optional mixture of two or more thereof can be used. Additionally, various lactoferrins produced in a microorganism, an animal cell or an animal using a gene engineering technique may also be used. In the present invention, hydrolyzed lactoferrin can also be used. These lactoferrins and lactoferrin derivatives which are commercially available may be used.

A molecular weight of lactoferrin is preferably about 1,000 to 200,000. Lactoferrin contained in the liposome includes hydrolyzed lactoferrin, and this hydrolyzed lactoferrin is one of preferable aspects. A content of lactoferrin contained in liposome is typically about 10 to 99% by weight, preferably about 20 to 95% by weight and more preferably about 30 to 90% by weight.

Lecithin includes, but is not limited to, for example, egg yolk lecithin, soybean lecithin, rapeseed lecithin, corn lecithin, sunflower lecithin and peanut lecithin, and can be used alone or in combination of two or more. In the present invention, hydrogenated lecithin can also be used. Lecithin is also referred to as phosphatidyl choline or 1,2-diacylglycerol-3-phosphocholine, and generally fatty acids are bound to positions 1 and 2 of glycerol. In the present invention, it is preferable to use lecithin in which an unsaturated fatty acid having 12 to 24 carbon atoms has been bound to both or either of the positions 1 and/or 2. It is particularly preferable to use lecithin in which a saturated fatty acid having 12 to 24 carbon atoms has bee bound to the position 1 and the unsaturated fatty acid having 12 to 24 carbon atoms has been bound to the position 2. Here, the saturated fatty acid and the unsaturated fatty acid may be straight or branched. As the preferable unsaturated fatty acid, the unsaturated fatty acid having 16 to 18 carbon atoms can be used. Particularly, lecithin in which oleic acid and linoleic acid have been abundantly bound to the position 2 is preferable. Specifically, egg yolk lecithin and soybean lecithin are preferable.

Sterol includes sterol derived from animals, such as cholesterol, lanosterol, dihydrolanosterol, desmosterol and dihydrocholesterol; sterol (phytosterol) derived from plants, such as β-sitosterol, campesterol, stigmasterol, brassicasterol, ergosterol, ergostadienol, sitosterol and brassicasterol; and sterol derived from microorganisms, such as zymosterol and ergosterol, and can be used alone or in combination of two or more. Among them, cholesterol or phytosterol is preferably used.

A molar ratio of lecithin to sterol in the liposome is preferably about 55:45 to 95:5, more preferably about 60:40 to 90:10, and most preferably about 75:25 to 85:15. When the molar ratio falls into this range, a stability of the liposome membrane is enhanced.

The content of lecithin in the liposomal lactoferrin is typically about 1 to 80% by weight, preferably about 3 to 65% by weight and more preferably about 5 to 50% by weight.

The content of sterol in the liposomal lactoferrin is typically about 0 to 40% by weight, preferably about 0.1 to 30% by weight and more preferably about 1 to 20% by weight.

The content of lecithin or sterol can be measured by known methods. For example, the content of lecithin can be quantified by Fiske-Subbarow method, and the content of sterol can be quantified by an HPLC or a colorimetric method.

Furthermore, the surface of the liposome containing lactoferrin can be coated, and this coating can also be utilized as an active component. The preferable coating includes coating with sulfuric acid group-containing polysaccharides. The sulfuric acid group-containing polysaccharides include fucoidan, carrageenan, agar and heparin. The sulfuric acid group-containing polysaccharides include sulfated polysaccharide containing no sulfuric acid group, and may be, for example, chondroitin sulfate and dermatan sulfate.

The molecular weight of the sulfuric acid group-containing polysaccharides is preferably about 5,000 to 300,000. Among these the sulfuric acid group-containing polysaccharides, fucoidan and carrageenan are preferably used, and particularly fucoidan is preferable.

The amount of the sulfuric acid group-containing polysaccharides to be used is preferably about 10 to 500 parts by weight and more preferably about 20 to 200 parts by weight relative to 100 parts by weight of lecithin contained in the liposome.

The coating can be performed, for example by adding the sulfuric acid group-containing polysaccharides to the suspension containing the liposomal lactoferrin and stirring at about 1000 to 3000 rpm for about 2 to 5 minutes. Multiple liposomes may be contained in one coating film.

The coating of the liposome with the sulfuric acid group-containing polysaccharides can be confirmed by the change of zeta potential of the liposome solution by adding the sulfuric acid group-containing polysaccharides and stirring.

In the liposomal lactoferrin, it is possible to if necessary add antioxidants such as tocopherol and ascorbic acid, organic acids such as lactic acid and citric acid, lipids such as phosphatidyl glycerol and phosphatidyl ethanolamine, natural polymers such as chitosan, fucoidan and hyaluronic acid, synthetic polymers such as polyethylene glycol and carboxy vinyl polymers, carbohydrates such as trehalose, lactulose and maltitol, and polyol such as glycerine in addition to lecithin and phytosterol.

In the liposomal lactoferrin, it is possible to if necessary encapsulate various substances as ingredients in addition to lactoferrin. These substances are, for example, chloramphenicol, fradiomycin sulfate, edetate sodium/cetrimide, epidihydrocholesterin, zinc chloride, dequalinium chloride, benzethonium chloride, dexamethasone, triamcinolone acetonide, sodium fluoride, non-steroidal anti-inflammatory drugs, adrenal cortex steroid drugs, sodium aurothiomalate, auranofin, penicillamine, bucillamine, lobenzarit disodium, salazosulfapyridine, actarit, methotrexate, mizoribine, anti-TNF antibody, soluble TNF receptor, anti-interleukin-1 antibody, anti-interleukin-6 antibody, estrogen formulations such as estradiol and estriol, raloxifene hydrochloride, bisphosphonate formulations such as alendronate, etidronate and risedronate, elcatonin, calcitonin, and ipriflavone.

The content of the liposomal lactoferrin in the osteoclast growth inhibitor, the oral composition and the preventive or therapeutic agent of the present invention is appropriately set depending on dosage forms, and is typically about 0.5 to 95% by weight, preferably about 0.8 to 80% by weight and more preferably about 1 to 50% by weight.

The osteoclast growth inhibitor of the present invention can be combined in the oral composition. Among the oral compositions, it is particularly preferable to utilize as a food composition or a pharmaceutical composition.

When the osteoclast growth inhibitor of the present invention is utilized as the food composition, it can be prepared using ordinary methods by combining the liposome with an edible carrier, a food material and a food additive as needed depending on the food form. As the food form, liquid foods such as beverages, and solid foods such as tablets, granules and chewable tablets can be utilized. It is also possible to utilize as semisolid foods such as yogurt. The specific food forms include liquid beverages such as juices, soft drinks and teas; powder beverages such as powder juices and powder soups; snacks such as chocolates, candies, chewing gums, ice creams, jellies, cookies, biscuits, cornflakes, chewable tablets, film sheets, wafers, gumi, rice crackers and steamed bean-jam bun; seasonings such as dressings and sauces; breads, noodles, alimentary yam pastes, paste foods (e.g., steamed fish pastes), various fish and vegetables flakes to sprinkle on cooked rice, sprays for oral cavity and lozenges. As additives, alive bacteria such as lactic acid bacteria or killed bacteria, other probiotics materials, vitamins, galenicals, plants such as herbs and extracts thereof may be combined.

The oral food compositions of the present invention can be used for intended uses of healthy foods, functional foods, foods for specified health use, foods with nutrient function claims, and foods for the sick for the prevention or the treatment of the bone diseases, for the prevention or the treatment of the periodontal disease with alveolar bone resorption, for the prevention or the treatment of the periodontal disease, for the prevention or the treatment of the periodontal disease with aging, for keeping the healthy teeth, for preventing loss of teeth, for the prevention or the treatment of the rheumatoid, for the prevention or the treatment of the osteoporosis or for keeping the healthy bone.

As the carrier of the oral food composition, for example, sugar alcohols such as maltitol, xylitol, sorbitol and erythritol; excipients such as crystalline cellulose, lactose, sucrose, glucose, starch, carbonate salts and phosphate salts; binders such as gelatin, alginic acid, xanthan gum, cellulose, hydroxypropylcellulose, methylcellulose, carrageenan, pullulan and pectin; emulsifiers such as sucrose fatty acid ester, sorbitan fatty acid ester, enzyme-treated lecithin, enzyme-degraded lecithin and saponin; antioxidants such as ascorbic acid and tocopherol; acidifiers such as lactic acid, citric acid, gluconic acid and glutamic acid; enhancement agents such as vitamins, amino acids, lactate salts, citrate salts and gluconate salts; fluidizers such as silicon dioxide; lubricants such as sucrose fatty acid ester and stearate salts; oligosaccharides such as lactulose, raffinose, fluctooligosaccharide, isomaltooligosaccharide, xylooligosaccharide and soybean oligosaccharide; dietary fibers such as indigestible dextrin, psylium and beet fibers; sweeteners and perfumes such as sucralose, acesulfame potassium, aspartame and glycyrrhizin can be used.

The content of the liposomal lactoferrin in the oral food composition is appropriately set depending on the dosage form of the composition, and is typically about 0.5 to 95% by weight, preferably about 0.8 to 80% by weight and more preferably about 1 to 50% by weight based on the total amount of the oral food composition.

When the osteoclast growth inhibitor of the present invention is utilized as the pharmaceutical composition, it is possible to use as the pharmaceutical composition in the form capable of being orally administered, such as liquid agents, solid agents such as tablets, granules, fine granules and powders, or capsules in which the liquid agent or the solid agent has been enclosed, by combining the pharmaceutically acceptable carrier as needed with the liposomal lactoferrin. The pharmaceutically acceptable carrier includes excipients and diluents. The pharmaceutical composition can also comprise various additives such as perfumes. Such a pharmaceutical composition can be used as the preventive or therapeutic agent for the bone diseases.

As the carrier, for example, excipients such as maltitol, lactose, sucrose, sodium chloride, glucose, starch, calcium carbonate, kaolin, crystalline cellulose, silic acid, methylcellulose, glycerine, sodium alginate, gum arabic, talc, calcium monohydrogen phosphate, calcium hydrogen phosphate, sodium hydrogen phosphate, dipotassium phosphate, potassium dihydrogen phosphate, calcium dihydrogen phosphate, sodium dihydrogen phosphate, calcium sulfate, calcium lactate and cacao butter; binders such as simple syrup, a glucose solution, a starch solution, a gelatin solution, polyvinyl alcohol, polyvinyl ether, polyvinyl pyrrolidone, cross polyvinyl pyrrolidone, hydroxypropylcellulose, low substitution hydroxypropylcellulose, hydroxypropylmethylcellulose, hydroxyethylcellulose, carboxyvinyl polymers, crystalline cellulose, powder cellulose, crystalline cellulose/carmelose sodium, carboxymethylcellulose, shellac, methylcellulose, ethylcellulose, potassium phosphate, gum arabic powder, pullulan, dextrin, maize starch, alpha starch, hydroxypropyl starch, gelatin, xanthan gum, tragacanth, tragacanth powder and macrogol; disintegrants such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch and lactose; disintegration inhibitors such as sucrose, stearic acid, cacao butter and hydrogenated oils; absorption accelerators such as quaternary ammonium salts and sodium lauryl sulfate; moisturizing agents such as glycerine and starch; absorbents such as starch, lactose, kaolin, bentonite and colloidal silic acid; and lubricants such as purified talc, stearate salts, boric acid powder and polyethylene glycol can be used. Furthermore, the tablets can be made into the tablets to which a usual coated film has been given as needed, e.g., sugar-coated tablets, gelatin-coated tablets, enteric coated tablets, film coating tablets, double tablets and multilayer tablets. The capsule is prepared by mixing the active component with various carriers exemplified above and filling the mixture in a hard gelatin capsule or a soft gelatin capsule.

The liquid formulation may be an aqueous or oily suspension, solution, syrup or elixir, and is prepared using the usual additives according to standard methods.

Examples of an aromatizing agent or a perfume include peppermint oil, eucalyptus oil, cinnamon oil, fennel oil, an oil of cloves, orange oil, lemon oil, rose oil, fruit flavors, mint flavors, peppermint powders, dl-menthol and 1-menthol.

The content of the liposomal lactoferrin in the oral pharmaceutical composition is appropriately set depending on the dosage form of the composition, and is typically about 0.5 to 95% by weight, preferably about 0.8 to 80% by weight and more preferably about 1 to 50% by weight based on the total amount of the oral pharmaceutical composition.

An ingestion dose (dosage) of the osteoclast growth inhibitor and the oral composition of the present invention is typically about 10 mg to 10,000 mg and preferably about 50 mg to 2,000 mg (when calculated in terms of lactoferrin) per day per adult in terms of ingested weight of the liposomal lactoferrin.

The osteoclast growth inhibitor and the oral composition of the present invention can be utilized in the fields where an osteoclast growth inhibiting action of the liposomal lactoferrin which is the active component is utilized, and for example, are useful for the prevention or the treatment of the bone diseases. In particular, since the osteoclast growth inhibitor of the present invention exhibits the osteoclast growth inhibiting action, it is useful for the diseases with the reduction of bone weight among the bone diseases. The diseases with the reduction of bone weight include osteoporosis, rheumatoid, osteoarthritis, periodontal diseases with alveolar bone resorption (gingivitis, periodontitis), wrong dental occlusion, physical tooth damage, alveolar bone resorption following the treatment of root canal, and the alveolar bone resorption due to oral blain or tumor.

### EFFECTS OF THE INVENTION

The osteoclast growth inhibitor and the oral composition of the present invention have the action to inhibit the growth of the osteoclast and are useful for the prevention or the treatment of the bone diseases.

### BEST MODES FOR CARRYING OUT THE INVENTION

The present invention will be described in more detail with reference to the following Examples and Test Examples, but the present invention is not limited thereto.

### EXAMPLES

### Example 1

### Preparation of liposomal lactoferrin (1)

A liposome formulation encapsulating lactoferrin was prepared by a thin film hydration method. Bovine lactoferrin was used. Egg yolk lecithin and phytosterol were weighted so that the molar ratio was 7:3 (egg yolk lecithin: 63.0 mg, phytosterol: 14.55 mg), and dissolved in 6 mL of ethanol. The solvent was distilled off from this solution using a rotary evaporator to form a thin film. Citrate buffer (6 mL, pH 6.69) in which 180 mg of lactoferrin had been dissolved was added to this thin film, which was then stirred at 2000 rpm for about 3 minutes using a vortex mixer to yield a liposome suspension. This suspension was filtrated through a polycarbonate film having a pore diameter of 0.4 µm to yield the liposomal lactoferrin. This liposome was negatively stained with 1% phosphotungstic acid, dried and then observed under an electron microscope to confirm that a multilamellar liposome was formed.

### Preparation of liposomal lactoferrin (2)

Egg yolk lecithin (1 g) and phytosterol (0.12 g) were weighted so that the molar ratio was 84:16, and dissolved in 5 mL of ethanol. 95 mL of an aqueous solution of 3% by weight of lactoferrin was added to this solution to preliminarily emulsify, and then high pressure homogenization was given to yield the liposomal lactoferrin. Bovine lactoferrin was used. This liposome was negatively stained with 1% by weight of phosphotungstic acid, dried and then observed under the electron microscope to confirm that the multilamellar liposome was formed.

### Preparation of liposomal lactoferrin (3)

A liposome formulation encapsulating lactoferrin and coated with fucoidan was prepared by the thin film hydration method. Bovine lactoferrin was used. Egg yolk lecithin and phytosterol were weighted (egg yolk lecithin: 63.0 mg, phytosterol: 14.55 mg) so that the molar ratio was 7:3, and dissolved in 6 mL of ethanol. The solvent was distilled off from this solution using the rotary evaporator to form a thin film. Citrate buffer (6 mL, pH 6.69) in which 180 mg of lactoferrin had been dissolved was added to this thin film, which was then stirred at 2,000 rpm for about 3 minutes using the vortex mixer to yield a liposome suspension. The liposome containing lactoferrin was yielded by filtration this suspension through the polycarbonate film having the pore diameter of 0.4 µm and dialysis the resulting filtrate using a cellulose ester membrane (MWCO 300,000) to remove free lactoferrin out of the liposome in the solution. Fucoidan (36 mg) was added to 6 mL of the resulting lactoferrin liposome suspension, which was then stirred strongly using the vortex mixer to yield the liposomal lactoferrin and coated with fucoidan.

### Preparation of liposomal lactoferrin (4)

A liposome containing lactoferrin was obtained in the same way as in the preparation of liposomal lactoferrin (1), except that bovine lactoferrin was changed to human lactoferrin.

### Preparation of liposomal lactoferrin (5)

A liposome enclosing lactoferrin was obtained in the same way as in the preparation of the liposomal lactoferrin (2), except that bovine lactoferrin was changed to human lactoferrin.

### Preparation of liposomal lactoferrin (6)

A liposome containing lactoferrin was obtained in the same way as in the preparation of the liposomal lactoferrin (3), except that bovine lactoferrin was changed to human lactoferrin.

### Preparation of liposomal lactoferrin (7)

A liposome containing lactoferrin was obtained in the same way as in the preparation of the liposomal lactoferrin (1), except that egg yolk lecithin was changed to soybean lecithin.

### Preparation of liposomal lactoferrin (8)

A liposome containing lactoferrin was obtained in the same way as in the preparation of the liposomal lactoferrin (2), except that egg yolk lecithin was changed to soybean lecithin.

### Preparation of liposomal lactoferrin (9)

A liposome containing lactoferrin was obtained in the same way as in the preparation of the liposomal lactoferrin (3), except that egg yolk lecithin was changed to soybean lecithin.

### Test Example 1

### Histological response by liposomal lactoferrin in rats topically applied with LPS

Wistar strain male rats aged 7 weeks were divided into a lactoferrin aqueous solution (MLF) group (30 rats), a lactoferrin-enclosed liposome dispersion (SLF) group (30 rats) and a control group (30 rats), and each sample to be tested was orally administered for 7 days. A dosage of lactoferrin per rat was 500 mg/kg/day. Only drinking water was given to the control group. Bovine lactoferrin and egg yolk lecithin were used.

### [Samples to be tested]

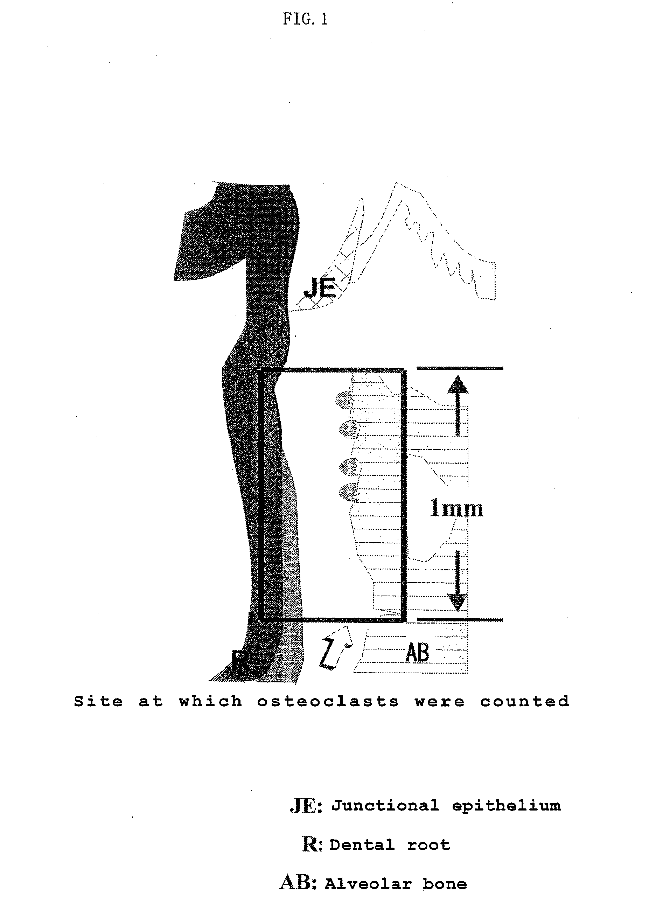

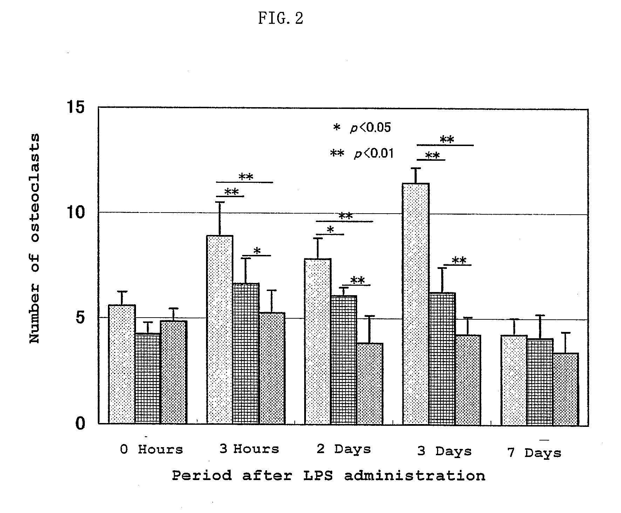

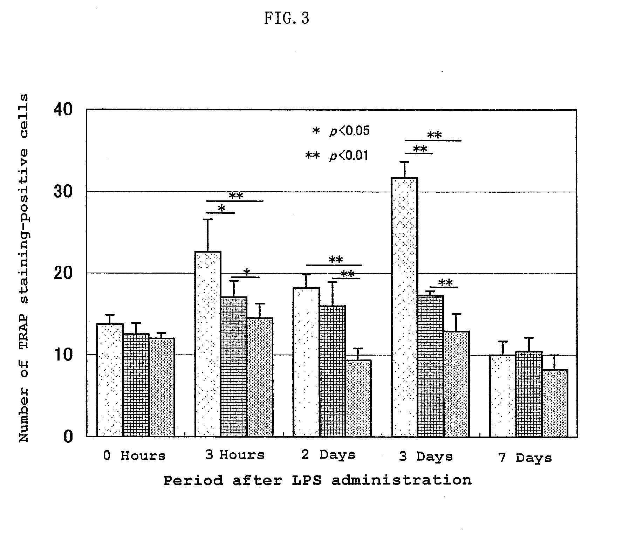

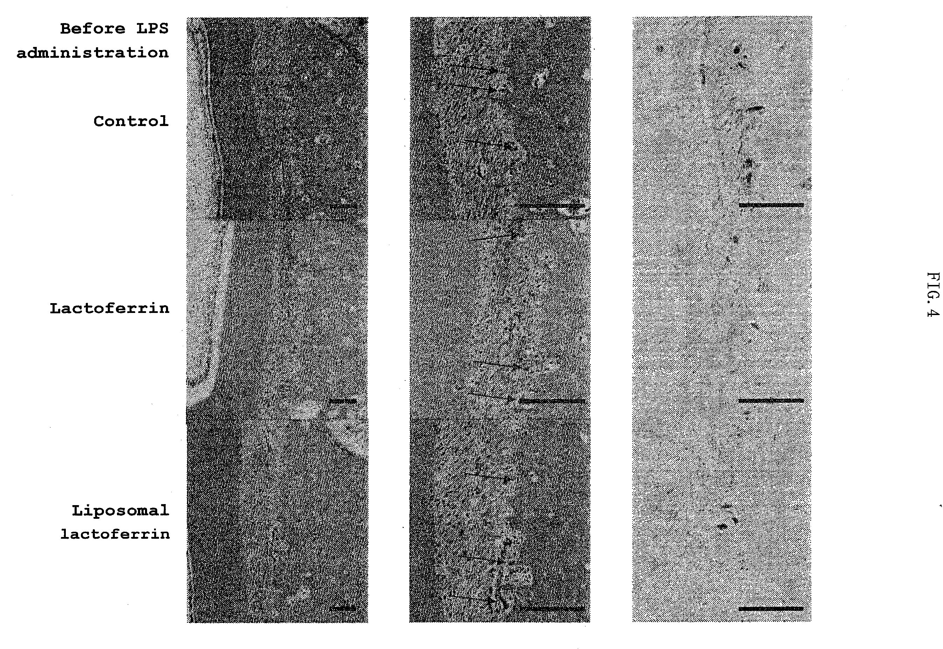

Control: drinking water
MLF: content of lactoferrin: 0.2125% by weight
SLF: content of lactoferrin: 0.2125% by weight, content of lecithin: 0.071% by weight, content of phytosterol: 0.0085% by weight
   Subsequently, a cotton plug impregnated with 5 mg/mL of LPS [lipopolysaccharide (osteoclast growth factor)] solution was left stand for one hour in a marginal periodontal tissue of a palate side of an upper jaw molar to infiltrate LPS from gingival troughs. Six rats were anatomized 0 hour (just before LPS administration), 3 hours, and 2, 3, and 7 days after the LPS administration, two sites of the marginal periodontal tissue per rat (n=12) were removed and the tissue was stained to count the number of polynuclear osteoclasts included in a region from an alveolar bone top to downward 1 mm (FIG. 1) in a periodontal membrane side of the alveolar bone. Specimen tissues were stained with hematoxylin eosin (HE) and tartaric acid-resistant acid phosphatase (TRAP). In HE staining, mononuclear cells are easily distinguished from polynuclear cells. In TRAP staining, the osteoclasts and precursor osteoclasts can be specifically stained with reddish color, and are easily distinguished from other cells. The numbers of the osteoclasts and TRAP staining-positive cells counted by both staining are shown in FIGs. 2 and 3. Among the TRAP staining positive cells, the polynuclear cell is the osteoclast and the mononuclear cell is the precursor osteoclast. The number of the TRAP staining positive cells corresponds to a sum total of the osteoclast number and the precursor osteoclast number. Microscopic photographs of the HE stained tissues after 0 hour, 3 hours and 3 days are shown in FIGs. 4 to 6. Each sample to be tested was continuously administered or ingested after the LPS administration. [Experimental condition]
   Experimental animals: Wistar strain male rats (7 weeks of age)
   Samples to be tested:
   lactoferrin (MLF): 500 mg/kg/day
   lactoferrin-encapsulated liposome (SLF): 500 mg/kg/day
   LPS solution: saline solution of LPS derived from *Escherichia coli* (5 mg/ml)
   Administration time period: administration of LPS by infiltration for one hour
   Observation time period: just before LPS administration, and 3 hours and 2, 3, 7 days after the start of the LPS administration
   Observation site: marginal periodontal tissue of palate side of upper jaw molar

As shown in FIG. 2, the osteoclasts grew in the control group after the LPS administration. In particular, the numbers of the osteoclasts were many after 3 hours and 3 days. The oral administration of lactoferrin not encapsulated in the liposome inhibited the increase of the osteoclasts in number (MLF group). However, the oral administration of lactoferrin encapsulated in the liposome further inhibited the increase of osteoclasts in number. In the comparison between MLF group and SLF group, although the dosages of lactoferrin in both groups were the same, the action to inhibit the increase of the osteoclasts in number was significantly high in SLF group. This tendency was similar in the numbers of the TRAP staining positive cells (FIG. 3). In the TRAP staining (FIG. 3), the numbers of the positive cells were decreased in SLF group, confirming that the increase of the osteoclast numbers was inhibited at a differentiation stage from the precursor osteoclast to the osteoclast.

### Test Example 2

A test was performed in the same way as in Test Example 1, except that six rats used per group and the dosage of the sample and the observation period were changed to the followings. The number of the rats anatomized in each observation period was two.

Lactoferrin- encapsulated liposome (S'LF): 100 mg/kg/day Observation period: just before LPS administration, and 3 hours and 3 days after the start of the LPS administration.

As a result of this test, in the comparison between MLF group and S'LF group, the actions to inhibit the increase of the osteoclasts in number were similar. The dosage of lactoferrin in S'LF group is 1/5 smaller than that in MLF group. Thus, it was shown that the lactoferrin-enclosed liposome gave the same action as that of lactoferrin alone by the smaller dosage.

### Production Example 1: Tablet

The following lecithin, sterol and lactoferrin (A components) were used to prepare a liposomal lactoferrin, and this liposome and the following other components (Component B) were used to produce tablets having the following composition by the standard methods.

| | |
|---|---|
| Egg yolk lecithin | 4.0% by weight (Component A) |
| Phytosterol | 1.0% by weight (Component A) |
| Lactoferrin | 15.0% by weight (Component A) |
| Maltitol | 42.0% by weight (Component B) |
| Lactose | 20.0% by weight (Component B) |
| Lactulose | 15.0% by weight (Component B) |
| Sucrose fatty acid ester | 3.0% by weight (Component B) |
| Total | 100% by weight |

### Production Example 2: Granule

The following lecithin and lactoferrin (Component A) were used to prepare a liposomal lactoferrin, and this liposome and the following other components (Component B) were used to produce granules having the following composition by the standard methods.

| | |
|---|---|
| Egg yolk lecithin | 5.0% by weight (Component A) |
| Lactoferrin | 20.0% by weight (Component A) |
| Lactose | 33.3% by weight (Component B) |
| Starch | 40.0% by weight (Component B) |
| Xanthan gum | 1.0% by weight (Component B) |
| Tocopherol | 0.1% by weight (Component B) |
| Gluconic acid | 0.1% by weight (Component B) |
| Perfume | 0.5% by weight (Component B) |
| Total | 100% by weight |

### Production Example 3: Beverage

The following lecithin, sterol and lactoferrin (Component A) were used to prepare a liposomal lactoferrin, and this liposome and the following other components (Component B) were used to produce a beverage having the following composition by the standard methods.

| | |
|---|---|
| Egg yolk lecithin | 0.5% by weight (Component A) |
| Phytosterol | 0.1% by weight (Component A) |
| Lactoferrin | 2.0% by weight (Component A) |
| Citric acid | 0.5% by weight (Component B) |
| Sodium citrate | 0.05% by weight (Component B) |
| Fructose/glucose solution | 10.0% by weight (Component B) |
| Vitamin C | 0.5% by weight (Component B) |
| Perfume | 0.15% by weight (Component B) |
| Purified water | remainder (component B) |
| Total | 100% by weight |

### Production Example 4: Chewable

The following lecithin, sterol and lactoferrin (Component A) were used to prepare a liposomal lactoferrin, and this liposome was coated with fucoidan (Component A), and this coated liposome and the following other components (Component B) were used to produce a chewable having the following composition by the standard methods.

| | |
|---|---|
| Soybean lecithin | 4.0% by weight (component A) |
| Phytosterol | 0.8% by weight (component A) |
| Lactoferrin | 40.0% by weight (component A) |
| Fucoidan | 1.4% by weight (component A) |
| Maltitol | 30.0% by weight (component B) |
| Xylitol | 20.0% by weight (component B) |
| Sucrose fatty acid ester | 3.0% by weight (component B) |
| Perfume | 0.8% by weight (component B) |
| Total | 100% by weight |

### INDUSTRIAL APPLICABILITY

The osteoclast growth inhibitor and the oral composition of the present invention can be utilized in the fields where the osteoclast growth inhibiting action of the liposomal lactoferrin which is the active component is utilized, and for example, can be utilized for the prevention or the treatment of the bone diseases due to the promotion of osteoclast growth.

### BRIEF DESCRIPTION OF THE DRAWINGS

FIG. 1 is a perpendicular sectional view showing sites where cell numbers were counted. JE, R and AB represent junctional epithelium, dental root and alveolar bone, respectively;
FIG. 2 is a graph representing the numbers of osteoclasts counted in Test Example 1, a horizontal axis denotes a time period after LPS administration (hours or days), a vertical axis denotes the number of osteoclasts, and three types of bars represent data from controls, a lactoferrin group and a lactoferrin-encapsulated liposome group from the left side (* p<0.05; **p<0.01);
FIG. 3 is a graph representing the numbers of TRAP staining-positive cells counted in Test Example 1, the horizontal axis denotes the time period after LPS administration (hours or days), the vertical axis denotes the number of TRAP staining-positive cells, and three types of bars represent data from the controls, the lactoferrin group and the lactoferrin-encapsulated liposome group from the left side (* p<0.05; **p<0.01);
FIG. 4 is a view showing microscopic photographs of periodontal tissues from rats 0 hour after LPS administration, observed and stained in Test Example 1 (each scale bar represents 100 µm), the left and central photographs show the periodontal tissues stained with HE, the right photographs show the periodontal tissues stained with TRAP, and the osteoclasts are shown by arrows in the central photographs;

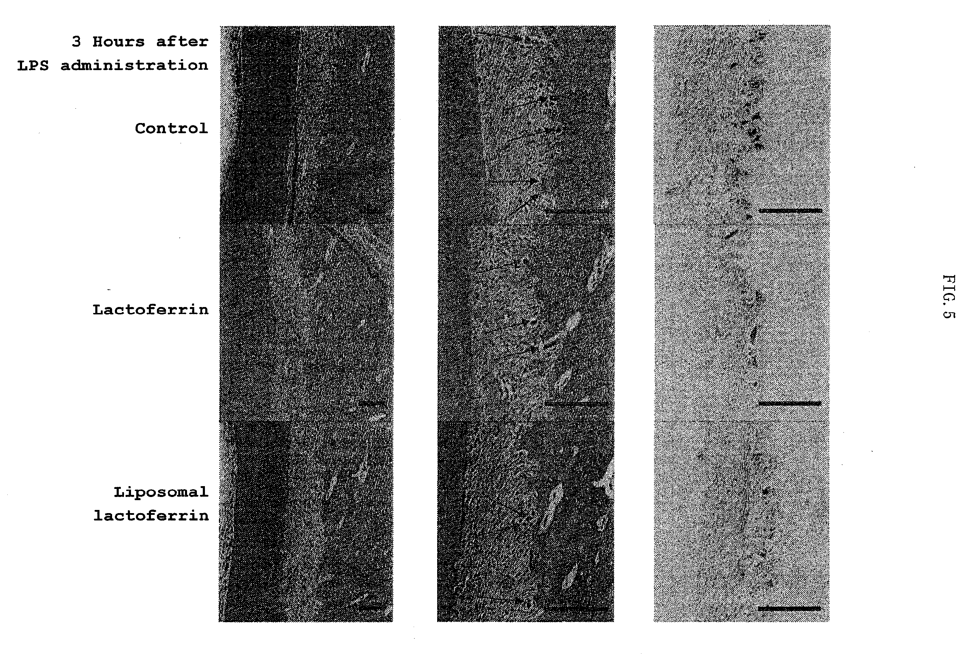

FIG. 5 is a view showing microscopic photographs of periodontal tissues of rats 3 hours after the LPS administration, observed and stained in Test Example 1 (each scale bar represents 100 µm), the left and central photographs show the periodontal tissues stained with HE, the right photographs show the periodontal tissues stained with TRAP, and the osteoclasts are shown by the arrows in the central photographs; and

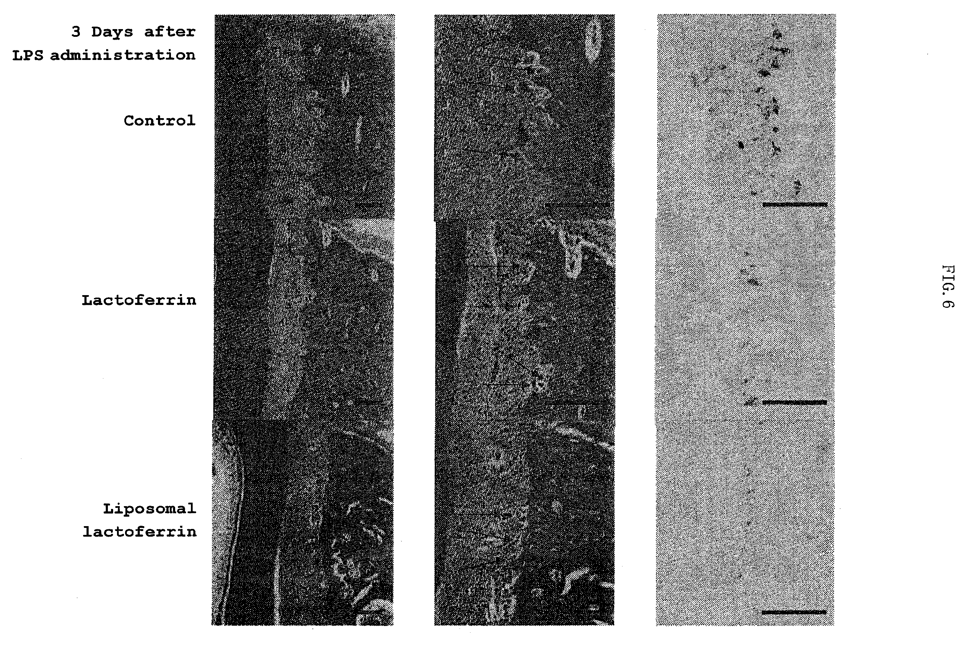

FIG. 6 is a view showing microscopic photographs of periodontal tissues of rats 3 days after the LPS administration, observed and stained in Test Example 1 (each scale bar represents 100 µm), the left and central photographs show the periodontal tissues stained with HE, the right photographs show the periodontal tissues stained with TRAP, and the osteoclasts are shown by the arrows in the central photographs.

## Claims

1. An osteoclast growth inhibitor containing a liposomal lactoferrin.

2. An oral composition containing the osteoclast growth inhibitor according to claim 1.

3. A preventive or therapeutic agent for bone diseases containing a liposomal lactoferrin.

4. The preventive or therapeutic agent according to claim 3 wherein the bone disease is osteoporosis.

5. The preventive or therapeutic agent according to claim 3 wherein the bone disease is rheumatoid.

6. The preventive or therapeutic agent according to claim 3 wherein the bone disease is a periodontal disease with alveolar bone resorption.

7. A preventive or therapeutic method for bone diseases wherein a liposomal lactoferrin in an amount effective for prevention or treatment of bone diseases is administered to a mammalian animal.

8. The preventive or therapeutic method according to claim 7 wherein the bone disease is osteoporosis.

9. The preventive or therapeutic method according to claim 7 wherein the bone disease is rheumatoid.

10. The preventive or therapeutic method according to claim 7 wherein the bone disease is a periodontal disease with alveolar bone resorption.

11. Use of a liposome containing lactoferrin for producing a preventive or therapeutic agent for bone diseases.

12. The use according to claim 11 wherein the bone disease is osteoporosis.

13. The use according to claim 11 wherein the bone disease is rheumatoid.

14. The use according to claim 11 wherein the bone disease is a periodontal disease with alveolar bone resorption.
